# EUROPEAN PATENT APPLICATION

(11) **EP 4 350 006 A1**
(43) Date of publication of application: **10.04.2024**
(21) Application number: 22815291.4
(22) Date of filing: 31.05.2022
(51) Int. Cl.: C12Q 1/686, C12Q 1/6827, C12Q 1/6886

(54) **NUCLEIC ACID COMBINATION FOR LIVER CANCER DIAGNOSIS OR AIDED DIAGNOSIS, TEST KIT, AND APPLICATION**

(30) Priority: 02.06.2021 CN 202110605465; 22.12.2021 CN 202111582152
(71) Applicant: Wuhan Ammunition Life-tech Co., Ltd., Wuhan, Hubei 430000 (CN)
(72) Inventor: ZHANG, Lianglu, Wuhan, Hubei 430000 (CN); ZHANG, Wei, Wuhan, Hubei 430000 (CN); DONG, Lanlan, Wuhan, Hubei 430000 (CN); LI, Guoqiang, Wuhan, Hubei 430000 (CN); ANG, Xue, Wuhan, Hubei 430000 (CN); LI, Tingting, Wuhan, Hubei 430000 (CN)
(74) Representative: Murgitroyd & Company
(86) International application number: PCT/CN2022/096490
(87) International publication number: WO 2022/253247

(57) **Abstract**

The present invention provides a nucleic acid combination for liver cancer diagnosis or aided diagnosis, a test kit, and an application. A CpG island site of a human GNB4 gene is used as a marker, or a GNB4 gene CpG site and a Riplet gene CpG site are used as markers. Liver cancer diagnosis or aid diagnosis is conducted by detecting the increase of the methylation level of the marker.

## Description

### CROSS-REFERENCE TO RELATED APPLICATIONS

This application claims the priority of Chinese patent application No. CN202110605465.X filed with the CNIPA on June 2, 2021, entitled "NUCLEIC ACID COMBINATION FOR LIVER CANCER DIAGNOSIS OR AUXILIARY DIAGNOSIS, DETECTION TEST KIT AND APPLICATION THEREOF", and Chinese patent application No. CN202111582152.3" filed with CNIPA on December 22, 2021, entitled "APPLICATION OF LIVER CANCER MARKERS IN PREPARING OF LIVER CANCER DETECTION PRODUCTS AND DETECTION KIT", the contents of which are incorporated herein by reference in their entireties.

### TECHNICAL FIELD

The present disclosure relates to the field of gene detection technologies, and in particular, to a combination of nucleic acids for diagnosis or auxiliary diagnosis of liver cancer, a test kit, and application thereof.

### BACKGROUND

Primary liver cancer is the fourth most common malignant tumor and the second most common tumor-lethal cause in China. Primary liver cancer mainly includes three different pathological types: hepatocellular carcinoma, intrahepatic cholangiocarcinoma, and combined hepatocellular carcinoma and intrahepatic cholangiocarcinoma. Hepatocellular carcinoma accounts for 85% to 90% of the primary liver cancer, and has risk factors including hepatitis B/ hepatitis C virus (HBV/HCV) infection, non-alcoholic fatty liver disease caused by obesity, chronic alcohol abuse, etc. These factors may directly lead to the development of cirrhosis. Patients with cirrhosis has an incidence rate of about 2% to 4% per year to develop into live cancer.

There are 855,000 new cases of primary liver cancer and 810,000 deaths due to primary liver cancer per year worldwide. The 5-year survival rate for patients with liver cancer is 30.1% in Japan, 27.2% in South Korea, and 17.4% in the United States. The prognosis of hepatocellular carcinoma is related to the severity of the disease at diagnosis. Because of late confirmation, only 25% of patients with newly diagnosed liver cancer can undergo surgical resection. 85% of patients with newly diagnosed liver cancer are already in the mid-to-late stage. Patients are often diagnosed when develop into the mid-to-late stage and lose the opportunity for surgery. Early screening, early diagnosis and early treatment, especially convenient, safe, fast and high-throughput screening methods, are effective ways to reduce the incidence and mortality of hepatocellular carcinoma.

Currently, there is a lack of sensitive and accurate methods for detection of hepatocellular carcinoma.

### SUMMARY

In an aspect, the present disclosure provides use of a reagent in preparing a kit for liver cancer diagnosis or auxiliary diagnosis. The reagent is used for detecting methylation of a target region in human GNB4 gene. The target region includes at least one CpG site and is selected from a full-length region or partial region of the following regions of GNB4 gene:
Chr3:179450970-179451662bp positive strand, and/or
Chr3:179451803-179451038bp negative strand.

The present disclosure also provides use of a reagent for liver cancer diagnosis or auxiliary diagnosis in preparing a liver cancer diagnosis or auxiliary diagnosis kit. The reagent is used for detecting a molecular marker. The molecular marker is a methylated CpG island of human GNB4 gene. The CpG island is selected from a full-length region or partial region of the following regions of GNB4 gene:
Chr3:179450970-179451662bp positive strand, and/or
Chr3:179451803-179451038bp negative strand.

In some embodiments, the target region is selected from a full-length region or partial region of at least one of the following regions, or the CpG island is selected from a full-length region or partial region of at least one of the following regions of GNB4 gene: a region 1, a region 2, a region 3, a region 4, a region 5, a region 6, a region 7, a region 8 and a region 9.

The region 1 is selected from Chr3: 179450970-179451058 bp positive strand, the region 2 is selected from Chr3: 179451090-179451203 bp positive strand, the region 3 is selected from Chr3: 179451231-179451356 bp positive strand, the region 4 is selected from Chr3: 179451379-179451472 bp positive strand, the region 5 is selected from Chr3: 179451444-179451662 bp positive strand, the region 6 is selected from Chr3: 179451803-179451709 bp negative strand, the region 7 is selected from Chr3: 179451675-179451569 bp negative strand, the region 8 is selected from Chr3: 179451457-179451278 bp negative strand, and the region 9 is selected from Chr3: 179451158-179451038 bp negative strand.

The present disclosure also provides a detection reagent including a nucleic acid combination. The nucleic acid combination is used for detecting methylation level of a target region in human GNB4 gene. The target region includes at least one CpG site and is selected from a full-length region or partial region of the following regions of GNB4 gene:
Chr3:179450970-179451662bp positive strand, and/or
Chr3:179451803-179451038bp negative strand.

The present disclosure also provides a detection reagent for liver cancer diagnosis or auxiliary diagnosis including a nucleic acid combination for detecting methylation level of a CpG island of human GNB4 gene. The CpG island is selected from a full-length region or partial region of the following regions of GNB4 gene:
Chr3:179450970-179451662bp positive strand, and/or
Chr3:179451803-179451038bp negative strand.

In some embodiments, the nucleic acid combination includes primers that can amplify the target region via PCR and/or probes that can label the target region.

In some embodiments, the target region or the CpG island is selected from a full-length region or partial region of at least one of the following regions of GNB4 gene: a region 1, a region 2, a region 3, a region 4, a region 5, a region 6, a region 7, a region 8 and a region 9.

The region 1 is selected from Chr3: 179450970-179451058 bp positive strand, the region 2 is selected from Chr3: 179451090-179451203 bp positive strand, the region 3 is selected from Chr3: 179451231-179451356 bp positive strand, the region 4 is selected from Chr3: 179451379-179451472 bp positive strand, the region 5 is selected from Chr3: 179451444-179451662 bp positive strand, the region 6 is selected from Chr3: 179451803-179451709 bp negative strand, the region 7 is selected from Chr3: 179451675-179451569 bp negative strand, the region 8 is selected from Chr3: 179451457-179451278 bp negative strand, and the region 9 is selected from Chr3: 179451158-179451038 bp negative strand.

In some embodiments, the nucleic acid combination is at least one selected from the group consist of: a nucleic acid combination 1 for detecting the region 1, a nucleic acid combination 2 for detecting the region 2, a nucleic acid combination 3 for detecting the region 3, a nucleic acid combination 4 for detecting the region 4, a nucleic acid combination 5 for detecting the region 5, a nucleic acid combination 6 for detecting the region 6, a nucleic acid combination 7 for detecting the region 7, a nucleic acid combination 8 for detecting the region 8, and a nucleic acid combination 9 for detecting the region 9.

The nucleic acid combination 1 has base sequences as set forth in SEQ ID NOs. 1 to 3, the nucleic acid combination 2 has base sequencesas set forth in SEQ ID NOs. 4 to 6, the nucleic acid combination 3 has base sequences as set forth in SEQ ID NOs. 7 to 9, the nucleic acid combination 4 has base sequences as set forth in SEQ ID NOs. 10 to 12, the nucleic acid combination 5 has base sequences as set forth in SEQ ID NOs. 13 to 15, the nucleic acid combination 6 has base sequences as set forth in SEQ ID NOs. 16 to 18, the nucleic acid combination 7 has base sequences as set forth in SEQ ID NOs. 19 to 21, the nucleic acid combination 8 has base sequences as set forth in SEQ ID NOs. 22 to 24, and the nucleic acid combination 9 has base sequences as set forth in SEQ ID NOs. 25 to 27.

In some embodiments, the detection reagent further includes a reagent for detecting a methylation status of the CpG island of human GNB4 gene. The means for detecting the methylation status includes at least one of: methylation-specific PCR, bisulfite sequencing, methylation-specific microarray, whole-genome methylation sequencing, pyrosequencing, methylation-specific high-performance liquid chromatography, digital PCR, methylation-specific high-resolution melting curve analysis, methylation-sensitive restriction enzyme digestion and flap endonuclease method.

The present disclosure also provides a detection kit for liver cancer diagnosis or auxiliary diagnosis, including any of the above detection reagents.

In some embodiments, a sample to be tested using the kit is a blood sample or tissue sample collected from an individual to be tested; and/or
the kit further includes a buffer, dNTPs, a DNA polymerase and water.

The present disclosure also provides a method for detecting, diagnosing, or assisting in diagnosis of, liver cancer, including:
A) detecting, in a sample from an individual to be tested, a methylation level of a full-length region or partial region in the following target regions of GNB4 gene:
   Chr3:179450970-179451662 bp positive strand, and/or
   Chr3:179451803-179451038 bp negative strand; and
B) analyzing a test result;
   wherein, the target region includes at least one CpG site.

In some embodiments, the target region is selected from a full-length region or partial region of at least one of the following regions of GNB4 gene: a region 1, a region 2, a region 3, a region 4, a region 5, a region 6, a region 7, a region 8 and a region 9.

The region 1 is selected from Chr3: 179450970-179451058 bp positive strand, the region 2 is selected from Chr3: 179451090-179451203 bp positive strand, the region 3 is selected from Chr3: 179451231-179451356 bp positive strand, the region 4 is selected from Chr3: 179451379-179451472 bp positive strand, the region 5 is selected from Chr3: 179451444-179451662 bp positive strand, the region 6 is selected from Chr3: 179451803-179451709 bp negative strand, the region 7 is selected from Chr3: 179451675-179451569 bp negative strand, the region 8 is selected from Chr3: 179451457-179451278 bp negative strand, and the region 9 is selected from Chr3: 179451158-179451038 bp negative strand.

In some embodiments, for the detection in step A), a reagent specific for the methylation of the target region of human GNB4 gene is used.

In some embodiments, the reagent includes a nucleic acid combination for detecting the methylation of the target region in human GNB4 gene.

In some embodiments, the nucleic acid combination includes primers that can amplify the target region via PCR and/or probes that can label the target region.

In some embodiments, the nucleic acid combination is at least one selected from the group consist of: a nucleic acid combination 1 for detecting the region 1, a nucleic acid combination 2 for detecting the region 2, a nucleic acid combination 3 for detecting the region 3, a nucleic acid combination 4 for detecting the region 4, a nucleic acid combination 5 for detecting the region 5, a nucleic acid combination 6 for detecting the region 6, a nucleic acid combination 7 for detecting the region 7, a nucleic acid combination 8 for detecting the region 8, and a nucleic acid combination 9 for detecting the region 9. The nucleic acid combination 1 has base sequences as set forth in SEQ ID NOs. 1 to 3, the nucleic acid combination 2 has base sequencesas set forth in SEQ ID NOs. 4 to 6, the nucleic acid combination 3 has base sequences as set forth in SEQ ID NOs. 7 to 9, the nucleic acid combination 4 has base sequences as set forth in SEQ ID NOs. 10 to 12, the nucleic acid combination 5 has base sequences as set forth in SEQ ID NOs. 13 to 15, the nucleic acid combination 6 has base sequences as set forth in SEQ ID NOs. 16 to 18, the nucleic acid combination 7 has base sequences as set forth in SEQ ID NOs. 19 to 21, the nucleic acid combination 8 has base sequences as set forth in SEQ ID NOs. 22 to 24, and the nucleic acid combination 9 has base sequences as set forth in SEQ ID NOs. 25 to 27.

In another aspect, the present disclosure also discloses use of a liver cancer marker in preparing a liver cancer detection product. The marker is a CpG site of GNB4 gene and a CpG site of Riplet gene.

In some embodiments, the CpG site of GNB4 gene is derived from at least one of the following regions: a region 10 and a region 11, wherein the region 10 is selected from Chr3:179450948-179451805 positive strand, and the region 11 is selected from Chr3:179451805-179450948 negative strand; and/or
the CpG site of Riplet gene is derived from at least one of the following regions: a region 12 and a region 13, wherein the region 12 is selected from Chr17:30971029-30971588 positive strand, and the region 13 is selected from Chr17:30971588-30971029 negative strand.

The present disclosure also provides a nucleic acid combination for liver cancer detection, including a nucleic acid combination 10 for detecting methylation of GNB4 gene and a nucleic acid combination 11 for detecting methylation of Riplet gene.

The nucleic acid combination 10 includes a pair of primers 1, the pair of primers 1 having base sequences that have at least 90% identity with a base sequence as set forth in SEQ ID NOs. 25-26.

The nucleic acid combination 11 includes a pair of primers 2, the pair of primers 2 having base sequences that have at least 90% identity with a base sequence as set forth in SEQ ID NOs. 42-43.

In some embodiments, the nucleic acid combination 10 further includes a probe 1, the probe 1 having a base sequence that has at least 90% identity with a base sequence as set forth in SEQ ID NO. 27.

The nucleic acid combination 11 further includes a probe 2, the probe 2 having a base sequence that has at least 90% identity with a base sequence as set forth in SEQ ID NO. 44.

The present disclosure also provides a kit, including the nucleic acid combination for liver cancer detection.

The present disclosure also provides use of a nucleic acid combination for liver cancer detection or a kit including the nucleic acid combination for liver cancer detection in detecting, diagnosing, or assisting in the diagnosis of, liver cancer.

The present disclosure also provides a method for detecting, diagnosing, or assisting in diagnosis of, liver cancer, including:
a) detecting, in a sample from an individual to be tested, methylation of a CpG site of GNB4 gene and methylation of a CpG site of Riplet gene; and
b) analyzing a test result.

In some embodiments, the CpG site of GNB4 gene is derived from at least one of the following regions: a region 10 and a region 11; wherein the region 10 is selected from Chr3:179450948-179451805 positive strand, and the region 11 is selected from Chr3:179451805-179450948 negative strand; and/or
the CpG site of Riplet gene is derived from at least one of the following regions: region 12 and region 13, wherein the region 12 is selected from Chr17:30971029-30971588 positive strand, and the region 13 is selected from Chr17:30971588-30971029 negative strand.

### DETAILED DESCRIPTION

In order to make the purpose, technical solutions, and advantages of the embodiments of the present disclosure clearer, the technical solutions in the embodiments of the present disclosure will be clearly and completely described below. If the specific conditions are not specified in the examples, they should be carried out according to the conventional conditions or the conditions recommended by the manufacturer. All reagents or instruments used without indicating the manufacturer are conventional products that can be purchased commercially.

DNA methylation is an early event in cancer development, and circulating tumor DNA (Ct DNA) in the blood is largely consistent with DNA in tumor tissue. Detecting methylation of Ct DNA in blood provides the possibility for early non-invasive detection of liver cancer. During carcinogenesis, abnormalities in DNA methylation levels often occur in CpG islands. CpG islands are mainly located in promoter and exon regions of a gene, which are regions rich in CpG dinucleotides, and have a length between 200-3000 bp and a G+C content higher than 50%.

In an aspect, the present disclosure provides a method for detecting, diagnosing, or assisting in the diagnosis of, liver cancer by detecting methylation of a CpG island of human GNB4 gene.

The present disclosure provides use of a reagent in preparing a kit for liver cancer diagnosis or auxiliary diagnosis. The reagent is used for detecting methylation of a target region in human GNB4 gene. The target region includes at least one CpG site and is selected from a full-length region or partial region of the following regions of GNB4 gene:
Chr3:179450970-179451662 bp positive strand, and/or
Chr3:179451803-179451038 bp negative strand.

An embodiment of the present disclosure provides use of a reagent for liver cancer diagnosis or auxiliary diagnosis in preparing a liver cancer diagnosis or auxiliary diagnosis kit. The reagent is used for detecting a molecular marker. The molecular marker is a methylated CpG island of human GNB4 gene. The CpG island is selected from a full-length region or partial region of the following regions of GNB4 gene:
Chr3:179450970-179451662 bp positive strand, and/or
Chr3:179451803-179451038 bp negative strand.

In the present disclosure, the methylated CpG island of human GNB4 gene is used as a marker to diagnose, or assist in the diagnosis of, liver cancer by detecting the increase in the methylation level of the CpG island of human GNB4 gene, which has high sensitivity and specificity and provides a new idea and option for the diagnosis of liver cancer. It can be understood that in the present disclosure, the target region of GNB4 gene to be detected includes a CpG site. Detecting the methylation of cytosine at the CpG site allows for diagnosis of, or assist in the diagnosis of, liver cancer. In some embodiments, the CpG site is a CpG island located in GNB4 gene. Of course, the CpG site can also be in regions other than the CpG island of GNB4 gene.

The detected region can be selected from a full-length region or partial region of Chr3:179450970-179451662bp positive strand and Chr3:179451803-179451038bp negative strand. Detecting the target region allows for diagnosis of, or assist in the diagnosis of, liver cancer.

It should be noted that the positions of the sites or regions mentioned in the present disclosure are based on the hg38 genome as the reference genome.

Chr3:179450970-179451662bp positive strand has a base sequence as set forth in SEQ ID NO.31, specifically as follows (5'-3'):

CACGCACGGGCTCGTGCTCTGAGTTCCTGGAAGGAGGCCTCGGGGAGTGACGAGAAACCAG
GGGGGTCTGCAGGACTTGGACCGCCGACCGTTCCTCGCTCCCCGGGGCGAGCGGTCTGGACCGCCCGGG
AAGTGCCTGCGCCGGCGGTCGTGGGGCCAGTTCCCGCGTGGCAGCTGGGCGCGACACAGGCGCGCCCTC
CTCGTCCCTCCCGGGCAGCGTCGGCCGCCCGAGCCCGGGGAGACCCGCCCCGCCCCGCGCCGTCACCCGG
GCCCCGTTCCGCAGGGGTGGCTCGCGGCGCCCCACGTCCCTGCGAGAAGCCCGGGATCGCTTCGCGGGGC
GCACCGACGAGCCGCCGCTCGCGAGCTCGCCGCCTACCTGGAGGGAGCTCAGGCCCGCGTCGACCGCGC
GCTGCCGGTGTCCGCTGGGCGCTCAGCAGCCCCTGGAGCGCGGAGCCGGCGTGGAGAGCGCAGCTCACA
GCCGAGACCAGAGCCGCCGGCCACACCCAGTCCCGCACCTCCCAGCAGCCAACTCCGCGGCGCGCCGGA
GCCGGGGCGGGGACGTGGCTGGAGGCGCGAGGCGCGAGGCACGAGGCGCGCGGGCCCGGCGGGGACGT
GCCGGGGACGCGCAGACCCTCGGAGCGCGCGCAGCCCGGGCGGGGGGCGAAGGGAGCGGGCGCCGCGC
GCAGCTTCTGC.

Chr3:179451803-179451038bp negative strand has a base sequence as set forth in SEQ ID NO.32, specifically as follows (5'-3'):

CCGGAAAGGAAACTGGCCACGCCACTTAGACGGGCGCTACTTAGCAGCGTGCCCCGGCGCC
ACACCAACAAGAAAACGAAACTGCCCGGGAATGAATGTTTTTCTTCTCCTTCTGGCAGGAAACGAGAGAG
AAAATAGGAAGCAGAAGCTGCGCGCGGCGCCCGCTCCCTTCGCCCCCCGCCCGGGCTGCGCGCGCTCCGA
GGGTCTGCGCGTCCCCGGCACGTCCCCGCCGGGCCCGCGCGCCTCGTGCCTCGCGCCTCGCGCCTCCAGC
CACGTCCCCGCCCCGGCTCCGGCGCGCCGCGGAGTTGGCTGCTGGGAGGTGCGGGACTGGGTGTGGCCG
GCGGCTCTGGTCTCGGCTGTGAGCTGCGCTCTCCACGCCGGCTCCGCGCTCCAGGGGCTGCTGAGCGCCC
AGCGGACACCGGCAGCGCGCGGTCGACGCGGGCCTGAGCTCCCTCCAGGTAGGCGGCGAGCTCGCGAGC
GGCGGCTCGTCGGTGCGCCCCGCGAAGCGATCCCGGGCTTCTCGCAGGGACGTGGGGCGCCGCGAGCCA
CCCCTGCGGAACGGGGCCCGGGTGACGGCGCGGGGCGGGGCGGGTCTCCCCGGGCTCGGGCGGCCGACG
CTGCCCGGGAGGGACGAGGAGGGCGCGCCTGTGTCGCGCCCAGCTGCCACGCGGGAACTGGCCCCACGA
CCGCCGGCGCAGGCACTTCCCGGGCGGTCCAGACCGCTCGCCCCGGGGAGCGAGGAACGGTCGGCGGTC
CAAGTCCTGCA.

In some embodiments of the present disclosure, the target region is selected from a full-length region or partial region of at least one of the following regions: a region 1, a region 2, a region 3, a region 4, a region 5, a region 6, a region 7, a region 8 and a region 9.

In some embodiments of the present disclosure, the above-mentioned CpG island is selected from a full-length region or partial region of at least one of the following regions of GNB4 gene: a region 1, a region 2, a region 3, a region 4, a region 5, a region 6, a region 7, a region 8 and a region 9.

The region 1 is selected from Chr3: 179450970-179451058 bp positive strand, the region 2 is selected from Chr3: 179451090-179451203 bp positive strand, the region 3 is selected from Chr3: 179451231-179451356 bp positive strand, the region 4 is selected from Chr3: 179451379-179451472 bp positive strand, the region 5 is selected from Chr3: 179451444-179451662 bp positive strand, the region 6 is selected from Chr3: 179451803-179451709 bp negative strand, the region 7 is selected from Chr3: 179451675-179451569 bp negative strand, the region 8 is selected from Chr3: 179451457-179451278 bp negative strand, and the region 9 is selected from Chr3: 179451158-179451038 bp negative strand.

In some embodiments, the target region is selected from a full-length region or partial region of at least one of the following regions: a region 2, a region 3, a region 8 and a region 9.

In some embodiments, the CpG island is selected from a full-length region or partial region of at least one of the following regions of GNB4 gene: a region 2, a region 3, a region 8 and a region 9.

It should be noted that in some embodiments, any one or more regions from the nine regions mentioned above can be selected for methylation detection as needed. For example, regions 1 to 5 are selected for methylation detection.

Based on the present disclosure, those skilled in the art can detect the methylation of one or more combinations of the above-mentioned regions by means of any technology known in the art to diagnose whether the subject has liver cancer. No matter what technology is used, it all falls within the protection scope of the present disclosure.

In some embodiments, the reagent for liver cancer diagnosis or auxiliary diagnosis is a reagent specific for the methylation of the target region of human GNB4 gene.

In some embodiments, the reagent for liver cancer diagnosis or auxiliary diagnosis is a nucleic acid combination specific for the methylation of the target region of human GNB4 gene.

In some embodiments, the nucleic acid combination specific for the methylation of the target region of human GNB4 gene includes primers and/or a probe specific for the methylation of the target region of human GNB4 gene.

In some embodiments, the nucleic acid combination for detecting the methylation of the target region of human GNB4 gene is at least one selected from the group consisting of: a nucleic acid combination 1 for detecting the region 1, a nucleic acid combination 2 for detecting the region 2, a nucleic acid combination 3 for detecting the region 3, a nucleic acid combination 4 for detecting the region 4, a nucleic acid combination 5 for detecting the region 5, a nucleic acid combination 6 for detecting the region 6, a nucleic acid combination 7 for detecting the region 7, a nucleic acid combination 8 for detecting the region 8, and a nucleic acid combination 9 for detecting the region 9. The nucleic acid combination 1 has base sequences as set forth in SEQ ID NOs. 1 to 3, the nucleic acid combination 2 has base sequencesas set forth in SEQ ID NOs. 4 to 6, the nucleic acid combination 3 has base sequences as set forth in SEQ ID NOs. 7 to 9, the nucleic acid combination 4 has base sequences as set forth in SEQ ID NOs. 10 to 12, the nucleic acid combination 5 has base sequences as set forth in SEQ ID NOs. 13 to 15, the nucleic acid combination 6 has base sequences as set forth in SEQ ID NOs. 16 to 18, the nucleic acid combination 7 has base sequences as set forth in SEQ ID NOs. 19 to 21, the nucleic acid combination 8 has base sequences as set forth in SEQ ID NOs. 22 to 24, and the nucleic acid combination 9 has base sequences as set forth in SEQ ID NOs. 25 to 27.

The present disclosure also provides a nucleic acid combination for detecting methylation level of a target region in human GNB4 gene. The target region includes at least one CpG site and is selected from a full-length region or partial region of the following regions of GNB4 gene:
Chr3:179450970-179451662 bp positive strand, and/or
Chr3:179451803-179451038 bp negative strand.

The present disclosure also provides a nucleic acid combination for detecting methylation level of a CpG island in human GNB4 gene. The CpG island is selected from a full-length region or partial region of the following regions of GNB4 gene:
Chr3:179450970-179451662 bp positive strand, and/or
Chr3:179451803-179451038 bp negative strand.

In some embodiments, the CpG island is selected from a full-length region or partial region of at least one of the following regions of GNB4 gene: a region 1, a region 2, a region 3, a region 4, a region 5, a region 6, a region 7, a region 8 and a region 9.

The region 1 is selected from Chr3: 179450970-179451058 bp positive strand, the region 2 is selected from Chr3: 179451090-179451203 bp positive strand, the region 3 is selected from Chr3: 179451231-179451356 bp positive strand, the region 4 is selected from Chr3: 179451379-179451472 bp positive strand, the region 5 is selected from Chr3: 179451444-179451662 bp positive strand, the region 6 is selected from Chr3: 179451803-179451709 bp negative strand, the region 7 is selected from Chr3: 179451675-179451569 bp negative strand, the region 8 is selected from Chr3: 179451457-179451278 bp negative strand, and the region 9 is selected from Chr3: 179451158-179451038 bp negative strand.

In some embodiments, the CpG island is selected from a full-length region or partial region of at least one of the following regions of GNB4 gene: a region 2, a region 3, a region 8 and a region 9. In plasma samples, the sensitivity of detection of methylation in the above four regions for liver cancer is greater than 85%, and the specificity of detection for liver cancer is greater than 95%. In liver tissure samples, the sensitivity of detection of methylation in the above four regions for liver cancer is greater than 95%, and the specificity of detection for liver cancer is greater than 100%.

In some embodiments, the nucleic acid combination for detecting the methylation of the target region of human GNB4 gene is a nucleic acid combination specific for the methylation of the target region of human GNB4 gene.

In some embodiments, the nucleic acid combination specific for the methylation of the target region of human GNB4 gene includes primers and/or a probe specific for the methylation of the target region of human GNB4 gene.

In some embodiments of the present disclosure, the nucleic acid combination for detecting methylation level of a CpG island in human GNB4 gene is at least one selected from the group consisting of: a nucleic acid combination 1 for detecting the region 1, a nucleic acid combination 2 for detecting the region 2, a nucleic acid combination 3 for detecting the region 3, a nucleic acid combination 4 for detecting the region 4, a nucleic acid combination 5 for detecting the region 5, a nucleic acid combination 6 for detecting the region 6, a nucleic acid combination 7 for detecting the region 7, a nucleic acid combination 8 for detecting the region 8, and a nucleic acid combination 9 for detecting the region 9.

The nucleic acid combination 1 has base sequences as set forth in SEQ ID NOs. 1 to 3, the nucleic acid combination 2 has base sequencesas set forth in SEQ ID NOs. 4 to 6, the nucleic acid combination 3 has base sequences as set forth in SEQ ID NOs. 7 to 9, the nucleic acid combination 4 has base sequences as set forth in SEQ ID NOs. 10 to 12, the nucleic acid combination 5 has base sequences as set forth in SEQ ID NOs. 13 to 15, the nucleic acid combination 6 has base sequences as set forth in SEQ ID NOs. 16 to 18, the nucleic acid combination 7 has base sequences as set forth in SEQ ID NOs. 19 to 21, the nucleic acid combination 8 has base sequences as set forth in SEQ ID NOs. 22 to 24, and the nucleic acid combination 9 has base sequences as set forth in SEQ ID NOs. 25 to 27.

The above-mentioned nucleic acid combinations are only one or more of the nucleic acid combinations provided by the applicant. In addition, in other embodiments, nucleic acid combinations as mentioned above with bases deleted or added are also within the protection scope of the present disclosure. For example, the nucleic acid combinations have 80% or more, e.g. more than 82%, more than 84%, more than 86%, more than 88%, more than 90%, more than 92%, more than 94%, more than 96%, more than 98% or more than 99% sequence identity to any nucleic acid combination of the above-mentioned nucleic acid combinations 1 to 9, are also within the protection scope of the present disclosure. For example, the nucleic acid combinations have 80% or more, e.g. more than 82%, more than 84%, more than 86%, more than 88%, more than 90%, more than 92%, more than 94%, more than 96%, more than 98% or more than 99% homology to any nucleic acid combination of the above-mentioned nucleic acid combinations 1 to 9, are also within the protection scope of the present disclosure.

In some embodiments of the present disclosure, the above-mentioned nucleic acid combination includes a probe sequence. The probe sequence is labeled with a fluorescent reporter group at 5' end, and a fluorescent quenching group at 3' end.

In some embodiments of the present disclosure, the above-mentioned fluorescent reporter group is HEX, FAM, TET, CF532, JOE, TAMRA, ROX, CY3, CY5, Texas Red, NED, Alexa Flour or VIC, and the quenching group is MGB, TAMRA, BHQ1, BHQ2, BHQ3 or QSY In other embodiments, the fluorescent reporter group and quenching group labeled on the probe can also be adaptively adjusted as needed and are not limited to the those defined above.

The present disclosure also provides a detection reagent for liver cancer diagnosis or auxiliary diagnosis including a nucleic acid combination for detecting methylation level of a CpG island of human GNB4 gene.

In some embodiments of the present disclosure, the above-mentioned detection reagent is a reagent for detecting a methylation level of the CpG island of human GNB4 gene. The means for detecting the methylation level includes at least one of: methylation-specific PCR, bisulfite sequencing, methylation-specific microarray, whole-genome methylation sequencing, pyrosequencing, methylation-specific high-performance liquid chromatography, digital PCR, methylations-specific high-resolution melting curve analysis, methylation-sensitive restriction enzyme digestion and flap endonuclease method.

The present disclosure also provides a detection kit for liver cancer diagnosis or auxiliary diagnosis, including a nucleic acid combination for detecting a CpG island of human GNB4 gene or a detection reagent for liver cancer diagnosis or auxiliary diagnosis.

In some embodiments of the present disclosure, a sample to be tested using the kit is a blood sample or tissue sample collected from an individual to be tested.

In some embodiments of the present disclosure, the kit further includes a buffer, dNTPs, a DNA polymerase and water.

An embodiment of the present disclosure provides use of the above-mentioned nucleic acid combination for detecting methylation level of the CpG island of human GNB4 gene, the above-mentioned detection reagent for liver cancer diagnosis or auxiliary diagnosis, or the above-mentioned detection kit for liver cancer diagnosis or auxiliary diagnosis in detecting, diagnosing, or assisting in the diagnosis of, liver cancer.

An embodiment of the present disclosure provides the above-mentioned nucleic acid combination for detecting methylation level of the CpG island of human GNB4 gene, the above-mentioned detection reagent for liver cancer diagnosis or auxiliary diagnosis, or the above-mentioned detection kit for liver cancer diagnosis or auxiliary diagnosis for use in detecting, diagnosing, or assisting in the diagnosis of, liver cancer.

An embodiment of the present disclosure also provides a method for detecting, diagnosing, or assisting in diagnosis of, liver cancer, including:
A) detecting, in a sample from a subject (an individual to be tested), a methylation level of a full-length region or partial region in the following target regions of GNB4 gene:
   Chr3:179450970-179451662 bp positive strand, and/or
   Chr3:179451803-179451038 bp negative strand; and
B) analyzing a test result,
   wherein, the target region includes at least one CpG site.

In some embodiments, for the detection in step A), the above-mentioned nucleic acid combination for detecting the methylation level of the target region of human GNB4 gene, or the above-mentioned detection reagent for liver cancer diagnosis or auxiliary diagnosis, or the above-mentioned detection kit for liver cancer diagnosis or auxiliary diagnosis can be used.

In some embodiments, step A) includes:
A1) extracting DNA from a sample of a subject (an individual to be tested);
A2) subjecting the extracted DNA to bisulfite conversion to afford converted DNA;
A3) detecting the converted DNA to determine the DNA methylation level in the sample.

When genomic DNA is subjected to bisulfite conversion, the cytosine in the 5'- CpG' -3 region remains intact if the DNA is methylated, or is converted to uracil if not methylated.

Step B) includes comparing the result of detecting the methylation level of the target region in the sample of the subject (the individual to be tested) with that in the sample of a normal individual who does not suffer from liver cancer, wherein the result of detection in the sample of the subject that is higher in methylation level than that in the sample of the normal individual who does not suffer from liver cancer indicates that the subject has liver cancer.

In some embodiments, the detection in step A) is achieved by: methylation-specific PCR, bisulfite sequencing, methylation-specific microarray, whole-genome methylation sequencing, pyrosequencing, methylation-specific high-performance liquid chromatography, digital PCR, methylations-specific high-resolution melting curve analysis, methylation-sensitive restriction enzyme digestion and flap endonuclease method.

In other aspect, the present disclosure provides a method for detecting, diagnosing, or assisting in the diagnosis of, liver cancer by detecting a CpG site of GNB4 gene and a CpG site of Riplet gene.

The present disclosure also disclose novel use of a liver cancer marker in preparing a liver cancer detection product. The marker is a CpG site of GNB4 gene and a CpG site of Riplet gene.

In other aspect, the present disclosure also provides use of a reagent in preparing a kit for liver cancer diagnosis or auxiliary diagnosis. The reagent is used for detecting a molecular marker. The molecular marker includes a CpG site of GNB4 gene and/or a CpG site of Riplet gene.

The present inventor have found that GNB4 gene or Riplet gene alone in liver cancer samples has sensitivity greater than 80% and specificity greater than 90% for detection, and further found that a combination of GNB4 gene and Riplet gene in liver cancer samples has sensitivity greater than 96% and specificity greater than 90% for detection. The sensitivity and specificity for detection of liver cancer with the combination of GNB4 gene and Riplet gene are higher than that with GNB4 + ACP1 combination, GNB4 + TSPYL1 combination, Riplet + ACP1 combination, Riplet + TSPYL1 combination, ACP1 + TSPYL1 combination, Riplet + TSPYL1 combination, Riplet + HOXA1 combination, Riplet+BMP4 combination, ACP1+TSPYL1 combination, ACP1+HOXA1 combination, ACP1+BMP4 combination, TSPYL1+HOXA1 combination, TSPYL1+BMP4 combination, and HOXA1+BMP4 combination. The combination of GNB4 gene and Riplet gene has synergistic effects and can greatly improve the sensitivity and specificity of liver cancer detection.

The above combination can effectively avoid false positives caused by interference from other liver diseases and has high specificity. The present disclosure provides a new idea for non-invasive diagnosis of liver cancer.

In an alternative embodiment, the methylation level of the above-mentioned marker can distinguish normal samples from primary liver cancer samples, and can also distinguish primary liver cancer from cirrhosis.

In some embodiments, the above-mentioned liver cancer detection product is at least one selected from the following products: a reagent, a kit, a chip and a sequencing library.

The reagent is in a form including but not limited to liquid, powder, granule, emulsion, and suspension.

In some embodiments, the above-mentioned CpG site of GNB4 gene is a nucleic acid molecule containing at least one CpG site located in at least one of the following CpG island regions: a region 10 and a region 11.

The region 10 is selected from Chr3:179450948-179451805 positive strand (SEQ ID NO. 57), and the region 11 is selected from Chr3:179451805-179450948 negative strand (the reverse complement of SEQ ID NO. 57).

In some embodiments, the above-mentioned CpG site of GNB4 gene is derived from at least one of the following regions: a region 10 and a region 11.

The region 10 is selected from Chr3:179450948-179451805 positive strand, and the region 11 is selected from Chr3:179451805-179450948 negative strand.

In some embodiments, the above-mentioned CpG site of Riplet gene is a nucleic acid molecule containing at least one CpG site located in at least one of the following CpG island regions: a region 12 and a region 13.

The region 12 is selected from Chr17:30971029-30971588 positive strand, and the region 13 is selected from Chr17:30971588-30971029 negative strand.

In some embodiments, the above-mentioned CpG site of Riplet gene is derived from at least one of the following regions: a region 12 and a region 13.

The region 12 is selected from Chr17:30971029-30971588 positive strand (SEQ ID NO. 58), and the region 13 is selected from Chr17:30971588-30971029 negative strand.

The target regions of the above markers are based on the hg38 genome as the reference genome. The inventor found that liver cancer tissues and blood samples have high methylation at CpG sites in the above region. By detecting the methylation level in this region, whether the sample is liver cancer can be determined.

In some embodiments, the reagent includes a reagent specific for the CpG site of human GNB4 gene and/or a reagent specific for the CpG site of human Riplet gene.

In some embodiments, the reagent includes a nucleic acid combination for the CpG site of human GNB4 gene and/or a nucleic acid combination specific for the CpG site of human Riplet gene.

In some embodiments, the reagent includes a first nucleic acid combination (a nucleic acid combination 10) for detecting methylation of GNB4 gene and/or a second nucleic acid combination (a nucleic acid combination 11) for detecting methylation of Riplet gene. The first nucleic acid combination includes a pair of primers 1, the pair of primers 1 having base sequences that have at least 90%, e.g. at least 91%, at least 92%, at least 93%, at least 94%, at least 95%, at least 96%, at least 97%, at least 98%, or at least 99% identity with base sequence as set forth in SEQ ID NOs. 25-26; and the second nucleic acid combination (i.e. the nucleic acid combination 11) includes a pair of primers 2, the pair of primers 2 having base sequences that have at least 90%, e.g. at least 91%, at least 92%, at least 93%, at least 94%, at least 95%, at least 96%, at least 97%, at least 98%, or at least 99% identity with base sequence as set forth in SEQ ID NOs. 42-43.

The present disclosure also provides a nucleic acid combination for liver cancer detection, including a first nucleic acid combination (a nucleic acid combination 10) for detecting methylation of GNB4 gene and a nucleic acid combination 11 for detecting methylation of Riplet gene;

The first nucleic acid combination (the nucleic acid combination 10) includes a pair of primers 1, the pair of primers 1 having base sequences that have at least 90% (e.g. 90% to 100%, 92% to 98%, 95% to 97%, such as 90%, 91%, 92%, 93%, 94%, 95%, 96%, 97%, 98%, 99%, 100%) identity with base sequence as set forth in SEQ ID NOs. 25-26.

The second nucleic acid combination (the nucleic acid combination 11) includes a pair of primers 2, the pair of primers 2 having base sequences that have at least 90% (e.g. 90% to 100%, 92% to 98%, 95% to 97%, such as 90%, 91%, 92%, 93%, 94%, 95%, 96%, 97%, 98%, 99%, 100%) identity with base sequence as set forth in SEQ ID NOs. 42-43.

In some embodiments, the first nucleic acid combination (the nucleic acid combination 10) further includes a probe 1, the probe 1 having a base sequence that has at least 90% (e.g. 90% to 100%, 92% to 98%, 95% to 97%, such as 90%, 91%, 92%, 93%, 94%, 95%, 96%, 97%, 98%, 99%, 100%) identity with a base sequence as set forth in SEQ ID NO. 27.

In some embodiments, the second nucleic acid combination (the nucleic acid combination 11) further includes a probe 2, the probe 2 having a base sequence that has at least 90% (e.g. 90% to 100%, 92% to 98%, 95% to 97%, such as 90%, 91%, 92%, 93%, 94%, 95%, 96%, 97%, 98%, 99%, 100%) identity with a base sequence as set forth in SEQ ID NO. 44.

The above nucleic acid sequences are as follows:
GNB4 forward primer: TTGTGTCGCGTTTAGTTGTTACG, SEQ ID NO. 25; GNB4 reverse primer: TACAAAACTTAAACCGCCGA, SEQ ID NO. 26; GNB4 probe: AATTGGTTTTACGATCGTCGGCGTA, SEQ ID NO. 27; Riplet forward primer: CTAGGACGATTTCGGTTGTATTATT, SEQ ID NO. 42 ; Riplet reverse primer: CTCCCTAACGACAAATAAAACAAAC, SEQ ID NO. 43; and Riplet probe: TTGTTGGATTGGTTCGTTACGTTGT, SEQ ID NO. 44.

In an alternative embodiment, the above-mentioned probe is labeled with a fluorescent reporter group at 5' end, and a fluorescent quenching group at 3' end. Fluorescent labeling may enable better signal amplification and high-sensitivity detection. In an embodiment, the above-mentioned probe is a Taqman probe.

The above-mentioned fluorescent reporter group is HEX, FAM, TET, CF532, JOE, TAMRA, ROX, CY3, CY5, Texas Red, NED, Alexa Flour or VIC, and the quenching group is MGB, TAMRA, BHQ1, BHQ2, BHQ3 or QSY.

An embodiment of the present disclosure also provides a kit, including the above-mentioned nucleic acid combination for liver cancer detection.

In some embodiments, the above-mentioned kit also includes one or more of a positive control, a negative control, detection primers for an internal reference gene, a detection probe for the internal reference gene, a DNA polymerase and a buffer.

In some embodiments, the above-mentioned internal reference gene is ACTB gene.

In some embodiments, a sample to be tested using the above-mentioned kit is a blood sample.

The blood sample is selected from a plasma sample, a serum sample, a whole blood sample or a blood cell sample.

An embodiment of the present disclosure also provides the above-mentioned nucleic acid combination for liver cancer detection for use in detecting, diagnosing, or assisting in the diagnosis of, liver cancer.

An embodiment of the present disclosure also provides a method for detecting, diagnosing, or assisting in diagnosis of, liver cancer, including:
a) detecting, in a sample from a subject (an individual to be tested) to be tested, methylation of a CpG site of GNB4 gene and methylation of a CpG site of Riplet gene; and
b) analyzing a test result.

In some embodiments, for detection in step a), the above-mentioned first nucleic acid combination (the nucleic acid combination 10) for detecting methylation of GNB4 gene and the above-mentioned second nucleic acid combination (the nucleic acid combination 11) for detecting methylation of Riplet gene, or a kit including the above-mentioned nucleic acid combination for liver cancer detection can be used.

In some embodiments, step a) includes:
a1) extracting DNA from a sample of a subject (an individual to be tested) to be tested;
a2) subjecting the extracted DNA to bisulfite conversion to afford converted DNA;
a3) detecting the converted DNA to determine the DNA methylation level in the sample.

When genomic DNA is subjected to bisulfite conversion, the cytosine in the 5'- CpG' -3 region remains intact if the DNA is methylated, or is converted to uracil if not methylated.

In some embodiments, the above-mentioned methylation level is detected by at least one of: methylation-specific PCR, sequencing, methylation-specific high-performance liquid chromatography, digital PCR, methylations-specific high-resolution melting curve analysis, methylation-specific microarray, methylation-sensitive restriction enzyme digestion and flap endonuclease method.

The above-mentioned sequencing is selected from the group consisting of bisulfite sequencing, whole-genome methylation sequencing, or pyrosequencing.

The above-mentioned flap endonuclease method is a fluorescent quantitative PCR that relies on Flap sequences produced by enzyme digestion. The flap endonuclease method usually includes three reactions, the reaction of FEN1 enzyme digestion to produce the Flap sequence, the PCR reaction, and the reaction of the Flap sequence binding to the FRET cassette to produce fluorescence. For details, please refer to US8361720B2 and Allawi,et al., "Invader Plus Method Detects Herpes Simplex Virus in Cerebrospinal Fluid and Simultaneously Differentiates Types 1 and 2", J Clin Microbiol., 2006,44:3443-7.

### Definition of Terms:

Herein, liver cancer includes three different types: hepatocellular carcinoma, intrahepatic cholangiocarcinoma, and combined hepatocellular carcinoma and intrahepatic cholangiocarcinoma. The above-mentioned markers can be used for early screening detection of the above three types of primary liver cancer.

As used herein, the term "methylation level" is the same as generally understood, and refers to whether cytosine in one or more CpG dinucleotides in a DNA sequence is methylated, or the frequency/ratio/percentage of methylation. It represents both qualitative and quantitative concepts. For example, if a cytosine (C) residue within a nucleic acid sequence is methylated, it may be referred to be "hypermethylated" or to have "increased methylation". In practical applications, different detection indicators can be used to compare DNA methylation levels according to actual situation. For example, in some cases, comparison can be made based on the Ct value measured for the samples. In some cases, the methylation ratios of the marker in the sample can be calculated according to: methylation ratio = number of methylated molecules/(number of methylated molecules + number of unmethylated molecules) × 100, and then compared. In some cases, the respective indicators needs to be subjected to statistical analysis and integration to obtain the final determination indicator.

As used herein, the term "positive strand" refers to the strand in the DNA double strand of a gene that carries the nucleic acid sequence encoding protein or amino acid information, also known as the coding strand or the sense strand. The nucleotide sequence of the positive strand is the same as the nucleic acid sequence of the mRNA.

As used herein, the term "negative strand" refers to the single strand of the DNA double strand of a gene that serves as a template for mRNA synthesis during transcription, also known as the template strand or the antisense strand.

Herein, the "subject" has the same meaning as and is used interchangeably with the "individual to be tested", refers to a vertebrate, preferably a mammal, most preferably a human, and refers to the object used for detection. The mammals include but are not limited to rodents, apes, humans, domestic animals, racer animals and pets. Tissues, cells and their progeny of biological entities obtained in vivo or cultured in vitro are also included.

Herein, the "nucleic acid sequence" has the same meaning as and is used interchangeably with the "base sequence", refers to the arrangement of bases in DNA or RNA, such as the arrangement of A, T, G, and C in DNA, or the arrangement of A, U, G, C in mRNA, rRNA, or tRNA.

The present disclosure has the following beneficial effects:

In the embodiments in which the detection, diagnosis, or, assistance in the diagnosis of, liver cancer, is based on detection of methylation of the CpG island of human GNB4 gene, the above-mentioned nucleic acid composition, detection reagent, and kit can be used to diagnose or assist diagnosis of liver cancer based on the methylation of the CpG island of human GNB4 gene as a marker by detecting the increased methylation level and have higher sensitivity and specificity. The detection reagents and kits provided in the present disclosure can effectively improve the true positive rate of liver cancer, thereby meeting the clinical needs for early screening and early diagnosis of hepatocellular carcinoma.

In the embodiments in which the detection, diagnosis, or, assistance in the diagnosis of, liver cancer, is based on detection of CpG site of GNB4 gene and CpG site of Riplet gene, a good marker is provided for early screening of liver cancer, and the marker composed of the combination of GNB4 gene and Riplet gene has synergistic effect and can greatly improve the sensitivity and specificity of liver cancer detection. The present disclosure has advantages, such as high early screening accuracy, non-invasion, convenience, safety, speediness and high throughput. The molecular markers provided in the present disclosure are expected to increase the proportion of early diagnosis of high-risk groups of primary liver cancer in China, allow early detection and early treatment, and reduce the incidence and mortality of primary liver cancer. The combination of GNB4 gene and Riplet gene can effectively avoid false positive caused by interference from other liver diseases and has high specificity. The present disclosure provides a new idea for non-invasive diagnosis of liver cancer.

### EXAMPLES

The present disclosure will be described in further detail with reference to following Examples.

### Example 1. Components of kits and detectable gene regions

In this example, a kit for liver cancer diagnosis or auxiliary diagnosis was provided. The kit includes a nucleic acid combination 1 including nucleic acid sequences as set forth in SEQ ID NOs.1-3, see Table 1 for sequences. This nucleic acid combination 1 can be used for detecting the methylation of Chr3: 179450970- 179451058 (the region 1) positive strand of GNB4 gene.

The region 1 positive strand has a base sequence as set forth in SEQ ID NO.33, specifically as follows (5'-3'):

CACGCACGGGCTCGTGCTCTGAGTTCCTGGAAGGAGGCCTCGGGGAGTGACGAGAAACCAG
GGGGGTCTGCAGGACTTGGACCGCCGAC.

### Example 2. Components of kits and detectable gene regions

In this example, a kit for liver cancer diagnosis or auxiliary diagnosis was provided. The kit includes a nucleic acid combination 2 including nucleic acid sequences as set forth in SEQ ID NOs.4-6, see Table 1 for sequences. This nucleic acid combination 2 can be used for detecting the methylation of Chr3: 179451090- 179451203 positive strand (the region 2) of GNB4 gene.

The region 2 positive strand has a base sequence as set forth in SEQ ID NO.34, specifically as follows (5'-3'):

ACCGCCCGGGAAGTGCCTGCGCCGGCGGTCGTGGGGCCAGTTCCCGCGTGGCAGCTGGGCG
CGACACAGGCGCGCCCTCCTCGTCCCTCCCGGGCAGCGTCGGCCGCCCGAGCC.

### Example 3. Components of kits and detectable gene regions

In this example, a kit for liver cancer diagnosis or auxiliary diagnosis was provided. The kit includes a nucleic acid combination 3 including nucleic acid sequences as set forth in SEQ ID NOs.7-9, see Table 1 for sequences. This nucleic acid combination 3 can be used for detecting the methylation of Chr3: 179451231- 179451356 positive strand (the region 3) of GNB4 gene.

The region 3 positive strand has a base sequence as set forth in SEQ ID NO.35, specifically as follows (5'-3'):

TCACCCGGGCCCCGTTCCGCAGGGGTGGCTCGCGGCGCCCCACGTCCCTGCGAGAAGCCCG
GGATCGCTTCGCGGGGCGCACCGACGAGCCGCCGCTCGCGAGCTCGCCGCCTACCTGGAGGGAGC.

### Example 4. Components of kits and detectable gene regions

In this example, a kit for liver cancer diagnosis or auxiliary diagnosis was provided. The kit includes a nucleic acid combination 4 including nucleic acid sequences as set forth in SEQ ID NOs. 10-12, see Table 1 for sequences. This nucleic acid combination 4 can be used for detecting the methylation of Chr3: 179451379- 179451472 positive strand (the region 4) of GNB4 gene.

The region 4 positive strand has a base sequence as set forth in SEQ ID NO.36, specifically as follows (5'-3'):

CTGCCGGTGTCCGCTGGGCGCTCAGCAGCCCCTGGAGCGCGGAGCCGGCGTGGAGAGCGCA
GCTCACAGCCGAGACCAGAGCCGCCGGCCACAC.

### Example 5. Components of kits and detectable gene regions

In this example, a kit for liver cancer diagnosis or auxiliary diagnosis was provided. The kit includes a nucleic acid combination 5 including nucleic acid sequences as set forth in SEQ ID NOs.13-15, see Table 1 for sequences. This nucleic acid combination 5 can be used for detecting the methylation of Chr3: 179451444- 179451662 positive strand (the region 5) of GNB4 gene.

The region 5 positive strand has a base sequence as set forth in SEQ ID NO.37, specifically as follows (5'-3'):

ACAGCCGAGACCAGAGCCGCCGGCCACACCCAGTCCCGCACCTCCCAGCAGCCAACTCCGC
GGCGCGCCGGAGCCGGGGCGGGGACGTGGCTGGAGGCGCGAGGCGCGAGGCACGAGGCGCGCGGGCCC
GGCGGGGACGTGCCGGGGACGCGCAGACCCTCGGAGCGCGCGCAGCCCGGGCGGGGGGCGAAGGGAGC
GGGCGCCGCGCGCAGCTTCTGC.

### Example 6. Components of kits and detectable gene regions

In this example, a kit for liver cancer diagnosis or auxiliary diagnosis was provided. The kit includes a nucleic acid combination 6 including nucleic acid sequences as set forth in SEQ ID NOs. 16-18, see Table 1 for sequences. This nucleic acid combination 6 can be used for detecting the methylation of Chr3: 179451803 - 179451709 negative strand (the region 6) of GNB4 gene.

The region 6 positive strand has a base sequence as set forth in SEQ ID NO.38, specifically as follows (5'-3'):

CCGGAAAGGAAACTGGCCACGCCACTTAGACGGGCGCTACTTAGCAGCGTGCCCCGGCGCC
ACACCAACAAGAAAACGAAACTGCCCGGGAATGA.

### Example 7. Components of kits and detectable gene regions

In this example, a kit for liver cancer diagnosis or auxiliary diagnosis was provided. The kit includes a nucleic acid combination 7 including nucleic acid sequences as set forth in SEQ ID NOs.19-21, see Table 1 for sequences. This nucleic acid combination 7 can be used for detecting the methylation of Chr3: 179451675- 179451569 negative strand (the region 7) of GNB4 gene.

The region 7 positive strand has a base sequence as set forth in SEQ ID NO.39, specifically as follows (5'-3'):

GAGAAAATAGGAAGCAGAAGCTGCGCGGCGCCCGCTCCCTTCGCCCCCGCCGGGCTG
CGCGCGCTCCGAGGGTCTGCGCGTCCCCGGCACGTCCCCGCCGGGC.

### Example 8. Components of kits and detectable gene regions

In this example, a kit for liver cancer diagnosis or auxiliary diagnosis was provided. The kit includes a nucleic acid combination 8 including nucleic acid sequences as set forth in SEQ ID NOs.22-24, see Table 1 for sequences. This nucleic acid combination 8 can be used for detecting the methylation of Chr3: 179451457- 179451278 negative strand (the region 8) of GNB4 gene.

The region 8 positive strand has a base sequence as set forth in SEQ ID NO.40, specifically as follows (5'-3'):

CTGGTCTCGGCTGTGAGCTGCGCTCTCCACGCCGGCTCCGCGCTCCAGGGGCTGCTGAGCGC
CCAGCGGACACCGGCAGCGCGCGGTCGACGCGGGCCTGAGCTCCCTCCAGGTAGGCGGCGAGCTCGCGA

GCGGCGGCTCGTCGGTGCGCCCCGCGAAGCGATCCCGGGCTTCTCGCAG.

### Example 9. Components of kits and detectable gene regions

In this example, a kit for liver cancer diagnosis or auxiliary diagnosis was provided. The kit includes a nucleic acid combination 9 including nucleic acid sequences as set forth in SEQ ID NOs.25-27, see Table 1 for sequences. This nucleic acid combination 9 can be used for detecting the methylation of Chr3: 179451158- 179451038 negative strand (the region 9) of GNB4 gene.

The region 9 positive strand has a base sequence as set forth in SEQ ID NO.41, specifically as follows (5'-3'):

CTGTGTCGCGCCCAGCTGCCACGCGGGAACTGGCCCCACGACCGCCGGCGCAGGCACTTCC
CGGGCGGTCCAGACCGCTCGCCCCGGGGAGCGAGGAACGGTCGGCGGTCCAAGTCCTGCA.

**Table 1. Sequence listing of the respective nucleic acid combinations**

| Ex. | Nucleic acid combination | Target gene | forward primer (5' - 3') | reverse primer (5' - 3') | Probe (5' - 3') |
|---|---|---|---|---|---|
| 1 | 1 | *GNB4* (positive strand region 1, SEQ ID NO33) | TACGTACGGGTTC GTGTTTTGA (SEQ ID NO.1) | ATCGACGATCCAA ATCCTACAA (SEQ ID NO.2) | TGGAAGGAGGT TTCGGGGAGT (SEQ ID NO.3) |
| 2 | 2 | *GNB4* (positive strand region 2, SEQ ID NO.34) | ATCGTTCGGGAAG TGTTTGC (SEQ ID NO.4) | AACTCGAACGAC CGACGCTA (SEQ ID NO.5) | TCGTGGGGTTA GTTTTCGCGT (SEQ ID NO.6) |
| 3 | 3 | *GNB4* (positive strand region 3, SEQ ID NO.35) | TTATTCGGGTTTC GTTTCG (SEQ ID NO.7) | ACTCCCTCCAAAT AAACGACGA (SEQ ID NO.8) | TTTACGTTTTG CGAGAAGTTCG G (SEQ ID NO.9) |
| 4 | 4 | *GNB4* (positive strand region 4, SEQ ID NO.36) | TTGTCGGTGTTCG TTGGGC (SEQ ID NO.10) | ATATAACCGACGA CTCTAATCTCGA (SEQ ID NO.11) | TAGTAGTTTTG GAGCGCGGAGT CG (SEQ ID NO.12) |
| 5 | 5 | *GNB4* (positive strand region 5, SEQ ID NO.37) | ATAGTCGAGATTA GAGTCGTCGGT (SEQ ID NO.13) | ACAAAAACTACG CGCGACG (SEQ ID NO.14) | TTAGTAGTTAAT TTCGCGGCGCG TC (SEQ ID NO.15) |
| 6 | 6 | *GNB4* (negative strand region 6, SEQ ID NO.38) | TCGGAAAGGAAA TTGGTTACG (SEQ ID NO.16) | TCATTCCCGAACA ATTTCGT (SEQ ID NO.17) | TTAGACGGCG TTATTTAGTAGC GT (SEQ ID NO.18) |
| 7 | 7 | *GNB4* (negative strand region 7, SEQ ID NO.39) | GAGAAAATAGGA AGTAGAAGTTGC G (SEQ ID NO.19) | ACCCGACGAAAA CGTACCGA (SEQ ID NO.20) | TTCGTTTTTCGT TCGGGTTGCG (SEQ ID NO.21) |
| 8 | 8 | *GNB4* (negative strand region 8, SEQ ID NO.40) | TTGGTTTCGGTTG TGAGTTGC (SEQ ID NO.22) | CTACGAAAAACC CGAAATCG (SEQ ID NO.23) | TTTAGGGGTTG TTGAGCGTTTA GCG (SEQ ID NO.24) |
| 9 | 9 | *GNB4* (negative strand | TTGTGTCGCGTTT AGTTGTTACG | TACAAAACTTAAA CCGCCGA | AATTGGTTTTAC GATCGTCGGCG |
| | | region 9, SEQ ID NO.41) | (SEQ ID NO.25) | (SEQ ID NO.26) | TA (SEQ ID NO.27) |

### Example 10. Composition of nucleic acid combination

In this example, a nucleic acid combination was provided, which includes the nucleic acid combination 2 in Example 2 and the nucleic acid combination 3 in Example 3.

### Example 11. Composition of nucleic acid combination

In this example, a nucleic acid combination was provided, which includes the nucleic acid combination 8 in Example 8 and the nucleic acid combination 9 in Example 9.

### Example 12. Composition of nucleic acid combination

In this example, a nucleic acid combination was provided, which includes the nucleic acid combination 2 in Example 2, the nucleic acid combination 3 in Example 3, the nucleic acid combination 8 in Example 8 and the nucleic acid combination 9 in Example 9.

### Example 13. Method for diagnosis of liver cancer using the kit

In this example, a method for diagnosing liver cancer using any one of the kits in Examples 1-9 was provided, which includes the following steps.

### (1) DNA extraction and bisulfite conversion

Blood sample: 5 mL of blood was centrifuged at 1300×g for 12 minutes to separate the plasma. The plasma was stored in a refrigerator at -80°C for later use. DNA in plasma was extracted using Magnetic bead serum/plasma cell-free DNA extraction kit (DP709) from Tiangen Biotech (Beijing) Co., Ltd. The extracted DNA was subjected to bisulfite conversion using EpiTech Bisulfite Kit according to the instructions of the kit. After conversion, unmethylated cytosine (C) was converted into uracil (U) while methylated cytosine remained unchanged. Uracil would be paired with adenine (A) and cytosine would be paired with Guanine (G) in the subsequent PCR step, thereby allowing for distinguishment between methylated and unmethylated sequences.

Tissue samples: For tissue samples, QIAamp DNA FFPE Tissue Kit (56404) was used to extract DNA according to the instructions of the kit.

### (2) Preparation of positive control and negative control

The positive control and negative control for each gene were artificially synthesized sequences constructed into vectors. The base composition of the artificially synthesized sequence was designed with reference to the sequence of the target fragment to be amplified. In the negative control, the sites corresponding to cytosines were all designed to be thymines. In the positive control, the sites corresponding to cytosines were designed to be thymines except for those corresponding to cytosines at the positions of CG dinucleotides. The nucleotides at other positions were the same as that in the target fragment to be amplified.

### (3) PCR reaction

Using β-actin as the internal reference gene, PCR reaction was performed in a system as shown in Table 2. β-actin is used as the internal reference gene. A forward primer for β-actin is: AAGGTGGTTGGGTGGTTGTTTTG (SEQ ID NO.28); and a reverse primer for β-actin is: AATAACACCCCCACCCTGC (SEQ ID NO.29). A probe for β-actin is: GGAGTGGTTTTTGGGTTTG (SEQ ID NO. 30).

In this example, the reporter group at 5 ' end of the probe for detecting the target region was FAM, and the quencher group at 3' end was MGB. The reporter group at 5' end of the β-actin probe was VIC, and the quencher group at 3' end was BHQ1.

**Table 2 PCR system**

| Components | Specification | Volume (µL) |
|---|---|---|
| Buffer | 5× | 5 |
| dNTPs | 2.5mM each | 2 |
| Forward primer for detection region | 10 µM | 1 |
| Reverse primer for detection region | 10 µM | 1 |
| Probe for detection region | 10 µM | 1 |
| Forward primer for β-actin | 10 µM | 1 |
| Reverse primer for β-actin | 10 µM | 1 |
| Probe for β-actin | 10 µM | 1 |
| DNA polymerase | 5U/µL | 0.5 |
| DNA from sample to be tested | / | 5 |
| Purified water | / | Replenish to 25 |

As shown in Table 2, when detecting the methylation status of any region from the region 1 to region 9 of GNB4 in the sample, the primers and probe corresponding to the region (that is, corresponding to the kit from Examples 1-9 of the present disclosure respectively), primers and probe for β-actin, buffer, dNTP, DNA polymerase and sample DNA were added at the volume in Table 2 into the reaction system.

PCR reaction conditions were shown in Table 3 below.

**Table 3 PCR reaction conditions**

| Step | Temperature | Time | Number of cycles | Fluorescence collection |
|---|---|---|---|---|
| Predenaturation | 95 °C | 10 min | 1 | No |
| Denaturation | 95 °C | 30 see | 45 | |
| Annealing | 60 °C | 30 see | | Yes |
| Supplementary extension | 72 °C | 5 min | 1 | |

Ct value reading: After PCR, the baseline was adjusted by setting the fluorescence value measured 1-2 cycles before the minimum Ct value of the sample in a PCR as the baseline value, the threshold was set at the inflection point of the S-type amplification curve, and the Ct value of each gene in the sample was obtained.

Quality control: the negative control and the positive control were tested simultaneously during each test. The negative control was purified water, and the positive control was a synthetic plasmid containing the β-actin gene and the target gene sequence and had a concentration of 10³ copies/microlitre. The negative control could not be amplified amplification, and the positive control would have an evident exponential growth phase with a Ct value between 26-30. The negative control, positive control and internal reference genes that meet the above requirements respectively would indicate that the test was valid and then could be used to determine the subsequent results for the samples. Otherwise, the test would be invalid and would be re-performed.

Results analysis and interpretation: when different samples were tested for methylation in the same region under the same experimental conditions, a smaller Ct value for the sample indicates a higher methylation level of the detected region in the sample. If a Ct value for a detection region in a sample was no more than 38, the detection region in the sample was considered to be methylation positive. If a Ct value for a detection region in a sample was more than 38, the detection region in the sample was considered to be methylation negative. The methylation test results of the sample were compared with the pathological results to calculate the sensitivity (i.e., sensitivity) and specificity for the methylation test. Sensitivity was the proportion of methylation-positive samples in samples with positive pathology results, and specificity is the proportion of methylation-negative samples in samples with negative pathology results.

### Experimental example 1. Validation of diagnostic tests

A total of 143 liver cancer tissue samples and 50 paracancerous samples were collected from Zhongnan Hospital of Wuhan University. According to the method described in Examples 13, the samples were subjected to genome extraction, and bisulfite conversion. Using the converted DNA as a template, the kits from Examples 1 to 9 of the present disclosure were respectively used for detecting the methylation status of the region 1 to the region 9. As shown in Table 4 below:

**Table 4. Sensitivity and specificity of the regions 1-9 for detecting liver tissue samples**

| | 143 liver cancer tissue samples | | 50 paracancerous tissue samples | |
|---|---|---|---|---|
| Detection region | Number of samples with positive PCR | Sensitivity | Number of samples with negative PCR | Specificity |
| region 1 | 127 | 88.81% | 50 | 100% |
| region 2 | 136 | 95.10% | 50 | 100% |
| region 3 | 138 | 96.50% | 50 | 100% |
| region 4 | 129 | 90.21% | 49 | 98% |
| region 5 | 128 | 89.51% | 50 | 100% |
| region 6 | 131 | 91.61% | 49 | 98% |
| region 7 | 130 | 90.91% | 48 | 96% |
| region 8 | 139 | 97.20% | 50 | 100% |
| region 9 | 140 | 97.90% | 50 | 100% |

As can be seen from the results in Table 4, in tissue samples, the detection sensitivities of regions 1-9 for liver cancer samples were above 85%, and the detection sensitivities of regions 2, 3, 8 and 9 were higher than the detection sensitivities of other regions and were all greater than 95%; and the detection specificities of regions 1-9 for liver cancer paracancerous tissues were all above 95%, and the detection specificities of regions 2, 3, 8 and 9 were all 100%.

### Experimental example 2. Validation of diagnostic tests

A total of 120 blood samples from healthy human and 95 blood samples from patients with liver cancer were collected from Zhongnan Hospital of Wuhan University. According to the method described in Examples 13, the blood samples were subjected to plasma separation, genome extraction, and bisulfite conversion. Using the converted DNA as a template, the kits from Examples 1 to 9 of the present disclosure were respectively used for detecting the methylation status of the region 1 to the region 9. Ct value = 38 was used as the critical value to calculate the sensitivity and specificity of the cancer samples and normal samples, as shown in Table 5 below:

**Table 5. Sensitivity and specificity of the regions 1-9 for detecting blood samples**

| | 95 plasma samples from patients with liver cancer | | 120 plasma samples from healthy human | |
|---|---|---|---|---|
| Detection region | Number of samples with positive PCR | Sensitivity | Number of samples with negative PCR | Specificity |
| region 1 | 79 | 83.16% | 117 | 97.50% |
| region 2 | 84 | 88.42% | 118 | 98.33% |
| region 3 | 86 | 90.53% | 119 | 99.17% |
| region 4 | 76 | 80.00% | 118 | 98.33% |
| region 5 | 77 | 81.05% | 118 | 98.33% |
| region 6 | 86 | 90.53% | 116 | 96.67% |
| region 7 | 79 | 83.16% | 117 | 97.50% |
| region 8 | 87 | 91.58% | 120 | 100.00% |
| region 9 | 88 | 92.63% | 118 | 98.33% |

As can be seen from the results in Table 5, in plasma samples, the detection sensitivities of regions 1-9 for liver cancer samples were above 80%, the detection sensitivities of regions 2, 3, 8 and 9 were higher than the detection sensitivities of other regions and were all greater than 85%, and the detection sensitivities of regions 3, 8 and 9 were greater than 90%; and the detection specificities of regions 1-9 for liver cancer paracancerous tissues were all above 95%, and the detection specificities of regions 2, 3, 8 and 9 were all greater than 98%.

### Example 14

In this example a nucleic acid combination for liver cancer detection was provided, which includes a first nucleic acid combination (a nucleic acid combination 10) for detecting methylation of GNB4 gene and a second nucleic acid combination (a nucleic acid combination 11) for detecting methylation of Riplet gene;

The first nucleic acid combination (nucleic acid combination 10) includes a pair of primers 1 (including forward primer and reverse primer) and a probe 1. The base sequences of the pair of primers 1 are as set forth in SEQ ID NOs. 25-26;

The first nucleic acid combination (nucleic acid combination 11) includes a pair of primers 2 and a probe 2. The base sequences of the pair of primers 2 are as set forth in SEQ ID NOs. 42-43.

The base sequence of the probe 1 is as set forth in SEQ ID NO.27, and the base sequence of the probe 2 is as set forth in SEQ ID NO.44.

The above nucleic acid sequences are as follows:
GNB4 forward primer: TTGTGTCGCGTTTAGTTGTTACG, SEQ ID NO. 25; GNB4 reverse primer: TACAAAACTTAAACCGCCGA, SEQ ID NO. 26; GNB4 probe: AATTGGTTTTACGATCGTCGGCGTA, SEQ ID NO. 27; Riplet forward primer: CTAGGACGATTTCGGTTGTATTATT, SEQ ID NO. 42; Riplet reverse primer: CTCCCTAACGACAAATAAAACAAAC, SEQ ID NO. 43; Riplet probe: TTGTTGGATTGGTTCGTTACGTTGT, SEQ ID NO. 44.

### Example 15

In this example, the methylation-specific PCR method was used for detecting the methylation level of genes in blood samples. The method specifically includes:

### (1) Extraction of blood cfDNA

Plasma cfDNA was extracted from 1.5 mL plasma sample using Magnetic bead serum/plasma cell-free DNA extraction kit (DP709) from Tiangen Biotech (Beijing) Co., Ltd according to the instructions of the kit. After the extraction, 50 µL purified water was used for elution.

### (2) Bisulfite conversion

The whole extracted genome was subjected to bisulfite conversion. The nucleic acid purification reagent (Recordation No. 20200843) from Wuhan Ammunition Life-tech Co.,Ltd was used as a nucleic acid conversion kit, and the experiment was implemented according to the instructions of the kit. After the conversion, 30 µL purified water was used for elution.

### (3) Methylation-specific PCR reaction

The bisulfite-converted DNA was subjected to methylation-specific PCR reaction to detect the methylation levels of the target genes GNB4, Riplet, ACP1, TSPYL5, HOXA1 and BMP4 in tissue samples and blood samples. Each gene was detected separately, that is, only the detection primers and probe for one gene were added to a PCR tube at a time, with the probe for detecting the internal reference gene ACTB. The sequences of forward and reverse primers and probes for each target gene are shown in table 6.

**Table 6 Primer and probe sequences for each target gene**

| Gene name | forward primer (5' - 3') | reverse primer (5' - 3') | probe (5' - 3') |
|---|---|---|---|
| *GNB4* | TTGTGTCGCGTTTAGTTGTTAC G (SEQ ID NO.25) | TACAAAACTTAAACCGCCG A (SEQ ID NO.26) | AATTGGTTTTACGATCGTC GGCGTA (SEQ ID NO.27) |
| *Riplet* | CTAGGACGATTTCGGTTGTATTA TT (SEQ ID NO.42) | CTCCCTAACGACAAATAAA ACAAAC (SEQ ID NO.43) | TTGTTGGATTGGTTCGTTA CGTTGT (SEQ ID NO.44) |
| *ACP1* | GCGCGTTGTTTCGTTTCG (SEQ ID NO.45) | CGTCACCTACCGCAAATAC G (SEQ ID NO.46) | CCACGGACGGCGGATAAG GAG (SEQ ID NO.47) |
| *TSPYL5* | GCGCGGGAGGATTTTCG (SEQ ID NO.48) | CCGCCACCATAAACGAACC (SEQ ID NO.49) | CCACGGACGCGAAATCGA AAT (SEQ ID NO.50) |
| *HOXA1* | GTACGTGACGGTGTTAGTTAAT GGT (SEQ ID NO.51) | ACGACGAAACGCCGAAAA T (SEQ ID NO.52) | AGTTTGTCGGTTTTCGTAG TGATGG (SEQ ID NO.53) |
| *BMP4* | TTCGTTGGAAGAAGTTTGTAGG (SEQ ID NO.54) | ACGCTTATTCCTACGATATC GTATC (SEQ ID NO.55) | ACGGGAATTTTTTTCGTCG GTTTC (SEQ ID NO.56) |
| *ACTB* | AAGGTGGTTGGGTGGTTGTTTT G (SEQ ID NO.28) | AATAACACCCCCACCTGC (SEQ ID NO.29) | GGAGTGGTTTTTGGGTTTG (SEQ ID NO.30) |

The probes used for detecting target genes were Taqman probes, in which the reporter group at 5' end was FAM and the quencher group at 3' end was MGB. For the internal reference gene ACTB probe, the reporter group at the 5' end of was VIC and the quencher group at the 3' end was BHQ1.

The components of the PCR reaction system are shown in Table 7.

**Table 7 Components of the PCR reaction system**

| Components | Specification | Volume (µL) |
|---|---|---|
| Taq Pro HS Probe Master Mix (Lot: QN111-01, with magnesium ion) | 2× | 25 |
| dNTP | dATP, dCTP, dTTP and dGTP, 2.5 mM each | 5 |
| Forward primer for target gene | 10 µM | 1.5 |
| Reverse primer for target gene | 10 µM | 1.5 |
| Probe for target gene | 10 µM | 1 |
| Forward primer for ACTB | 10 µM | 1.5 |
| Reverse primer for ACTB | 10 µM | 1.5 |
| Probe for ACTB | 10 µM | 1 |
| Hot start DNA polymerase | 5U/µL | 0.6 |
| Converted blood sample DNA | / | 10 |
| Purified water | / | Replenish to 50 |

PCR reaction conditions were shown in Table 8 below.

**Table 8 PCR reaction conditions**

| Step | Temperature | Time | Number of cycles | Fluorescence collection |
|---|---|---|---|---|
| Predenaturation | 95 °C | 10 min | 1 | No |
| Denaturation | 95 °C | 30 see | 50 | |
| Annealing and extension | 60 °C | 30 see | | Yes |

For negative control and positive control: when each target gene were detected separately, the negative control and positive control should be tested simultaneously during each test. The negative control was purified water. The positive control was a synthetic plasmid.

The preparation method of positive control was as follows. The bisulfite-converted sequences corresponding to fully methylated regions of each target gene to be amplified (in which the cytosines in each amplified region were designed to be thymines except for cytosines at the positions of CG dinucleotides, and the types and positions of the other three types of bases remain unchanged) were artificially synthesized and cloned into the vector pUC57 to form a synthetic plasmid. The plasmid was diluted to 10³ copies/microliter.

Ct value reading: After PCR, the baseline was adjusted by setting the fluorescence value measured 1-2 cycles before the minimum Ct value of the sample in a PCR as the baseline value, the threshold was set at the inflection point of the S-type amplification curve, and the Ct value of each gene in the sample was obtained.

Quality control: the negative control could not be amplified, and the positive control have a Ct value between 26-30. The internal reference gene of the sample to be tested have a Ct value that is no more than 33. The negative control, positive control and internal reference genes that meet the above requirements respectively would indicates that the test was valid and then could be used to determine the subsequent results for the samples. Otherwise, the test will be invalid and would be re-performed.

### (4) PCR result analysis

If a Ct value for a target gene in a sample is no more than 43, the target gene in the sample is considered to be methylation-positive. If a Ct value for a target gene in a sample is more than 43, the target gene in the sample will be considered to be methylation negative. When evaluating the detection effect for a combination of two genes, as long as at least one gene in the sample is methylation positive, the gene combination in the sample will be considered to be methylation positive, and if both genes in a sample are methylation negative, the gene combination in the sample will be considered to be methylation negative. The methylation test results of the sample were compared with the pathological results to calculate the sensitivity and specificity of the methylation test:

In this example, sensitivity was the proportion of methylation-positive samples in samples with positive pathology results, and specificity is the proportion of methylation-negative samples in samples with negative pathology results.

### Experimental example 3

A total of 118 blood samples from patients with liver cancer, 55 blood samples from patients with cirrhosis, and 191 blood samples from healthy human were collected from a hospital in Zhengzhou. The samples were subjected to plasma cfDNA extraction and bisulfite conversion according to the method in Example 15. The converted DNA was then used as a template to detect the methylation levels of the genes GNB4, Riplet, ACP1, TSPYL1, HOXA1 and BMP4. The number of samples with positive /negative PCR for the six genes in the three types of samples was counted to calculate the sensitivity and specificity. The results are shown in Table 9 below :

**Table 9 Statistical results for blood sample**

| Tested genes | 118 plasma samples from patients with liver cancer | | 55 plasma samples from patients with cirrhosis | | 191 plasma samples from healthy human | |
|---|---|---|---|---|---|---|
| | Number of PCR positives | Sensitivity | Number of PCR negatives | Specificity | Number of PCR negatives | Specificity |
| *GNB4* | 102 | 86.44% | 52 | 94.55% | 189 | 98.95% |
| *Riplet* | 96 | 81.36% | 53 | 96.36% | 188 | 98.43% |
| *ACP1* | 84 | 71.19% | 52 | 94.55% | 184 | 96.34% |
| *TSPYL1* | 99 | 83.90% | 50 | 90.91% | 188 | 98.43% |
| *HOXA1* | 73 | 61.86% | 53 | 96.36% | 183 | 95.81% |
| *BMP4* | 94 | 79.66% | 52 | 94.55% | 184 | 96.34% |

As could be seen from the results in Table 9, for 118 liver cancer samples and among the six genes, three genes, i.e. GNB4, Riplet and TSPYL1 all have sensitivity of greater than 80%, except for ACP1, HOXA1 and BMP4, which had detection sensitivity below 80%. GNB4 has the highest sensitivity, 86.44%. The specificity of the six genes was all greater than 90% for the cirrhosis samples. The specificity of the six genes, i.e. GNB4, Riplet, TSPYL1, ACP1 and BMP4 were all greater than 96% for 191 plasma samples from healthy human.

Furthermore, the ensitivity and specificity of the six genes were evaluated when the six genes were combined in pairs for detecting 118 blood samples from patients with liver cancer, 55 blood sample from patients with liver cirrhosis, and 191 blood samples from healthy human. The results are shown in Table 10.

**Table 10 Statistical results of sensitivity and specificity of the combinations of two genes for detecting blood samples**

| Tested genes | 118 plasma samples from patients with liver cancer | | 55 plasma samples from patients with cirrhosis | | 191 plasma samples from healthy people | |
|---|---|---|---|---|---|---|
| | Number of samples with positive PCR | Sensitivity | Number of samples with negative PCR | Specificity | Number of samples with negative PCR | Specificity |
| *GNB4*+*Riplet* | 114 | 96.61% | 52 | 94.55% | 187 | 97.91% |
| *GNB4*+*ACP1* | 105 | 88.98% | 51 | 92.73% | 182 | 95.29% |
| *GNB4*+*TSPYL1* | 108 | 91.53% | 47 | 85.45% | 186 | 97.38% |
| *GNB4*+*HOXA1* | 102 | 86.44% | 51 | 92.73% | 182 | 95.29% |
| *GNB4*+*BMP4* | 103 | 87.29% | 51 | 92.73% | 182 | 95.29% |
| *Riplet*+*ACP1* | 101 | 85.59% | 50 | 90.91% | 181 | 94.76% |
| *Riplet*+*TSPYL1* | 104 | 88.14% | 48 | 87.27% | 185 | 96.86% |
| *Riplet*+*HOXA1* | 98 | 83.05% | 52 | 94.55% | 182 | 95.29% |
| *Riplet*+*BMP4* | 100 | 84.75% | 50 | 90.91% | 181 | 94.76% |
| *ACP1*+*TSPYL1* | 106 | 89.83% | 49 | 89.09% | 181 | 94.76% |
| *ACP1*+*HOXA1* | 89 | 75.42% | 51 | 92.73% | 177 | 92.67% |
| *ACP1*+*BMP4* | 96 | 81.36% | 49 | 89.09% | 179 | 93.72% |
| *TSPYL1*+*HOXA1* | 100 | 84.75% | 48 | 87.27% | 181 | 94.76% |
| *TSPYL1*+*BMP4* | 102 | 86.44% | 47 | 85.45% | 182 | 95.29% |
| *HOXA1*+*BMP4* | 97 | 82.20% | 50 | 90.91% | 177 | 92.67% |

As can be seen from the results in Table 10, when two genes were combined, the sensitivity of 15 pairs of combinations is improved for testing 118 liver cancer samples. The sensitivity of two combinations, i.e. the combination of GNB4 with Riplet (GNB4+Riplet combination) and the combination of GNB4 with TSPYL1 (GNB4+TSPYL1 combination), was increased to more than 90%. The GNB4+Riplet combination had the most obviously improved sensitivity, i.e. 96.61%.

For the 55 liver cirrhosis samples, there were 9 combinations with specificity greater than 90%, including the GNB4+Riplet combination. For 191 blood samples from healthy human, the specificity of 15 combinations was all greater than 90%. Taken together, the GNB4+Riplet combination has better detection sensitivity and specificity.

In summary, for plasma samples, GNB4 gene has the best sensitivity and specificity for methylation detection, better than the five genes Riplet, ACP1, TSPYL 1, HOXA1 and BMP4. When GNB4 is combined with Riplet, the sensitivity can be significantly improved without reducing specificity. Therefore, the GNB4 and Riplet gene combination can be applied to detect the methylation of blood samples, and can improve the popularity of detection reagents. This disclosure provides a new idea for non-invasive detection and early screening of liver cancer.

The above are only alternative embodiments of the present disclosure, and are not intended to limit the present disclosure. For those skilled in the art, various modifications and variations may be made to the present disclosure. Any modifications, equivalent replacements, improvements, and the like made within the spirit and principles of this disclosure shall fall within the protection scope of this disclosure.

### Industrial applicability

The present disclosure provides a nucleic acid combination for liver cancer diagnosis or auxiliary diagnosis, a detection kit, and use thereof. In the present disclosure, a methylated CpG island of human GNB4 gene is used as a marker, or a CpG site of GNB4 gene and a CpG site of Riplet gene is used as a marker to diagnose, or assist in the diagnosis of, liver cancer by detecting the increased methylation level thereof. A methylated CpG island of human GNB4 gene has high sensitivity and specificity of detection. Meanwhile, the marker composed of the combination of GNB4 gene and Riplet gene not only has high sensitivity and specificity, but also has synergistic effect, which can effectively avoid false positive caused by interference from other liver diseases, has high specificity, and thus can greatly improve the sensitivity and specificity of liver cancer detection. Using the nucleic acid combinations, detection reagents or kits provided in the present disclosure can effectively improve the true positive rateof liver cancer, thereby meeting the clinical needs for early screening and early diagnosis of hepatocellular carcinoma. The nucleic acid combinations, detection reagents or kits provided in the present disclosure have excellent performance in application and can be widely used in the field of liver cancer diagnosis and auxiliary diagnosis.

## Claims

1. Use of a reagent in preparing a kit for liver cancer diagnosis or auxiliary diagnosis, wherein the reagent is used for detecting methylation of a target region in human GNB4 gene, and the target region comprises at least one CpG site and is selected from a full-length region or partial region of the following regions of GNB4 gene:
Chr3:179450970-179451662 bp positive strand, and/or
Chr3:179451803-179451038 bp negative strand.

2. Use of a reagent for liver cancer diagnosis or auxiliary diagnosis in preparing a liver cancer diagnosis or auxiliary diagnosis kit, wherein the reagent is used for detecting a molecular marker, the molecular marker is a methylated CpG island of human GNB4 gene, and the CpG island is selected from a full-length region or partial region of the following regions of GNB4 gene:
Chr3:179450970-179451662 bp positive strand, and/or
Chr3:179451803-179451038 bp negative strand.

3. The use according to claim 1 or 2, wherein the target region according to claim 1 is selected from a full-length region or partial region of at least one of the following regions, or the CpG island according to claim 2 is selected from a full-length region or partial region of at least one of the following regions of GNB4 gene: a region 1, a region 2, a region 3, a region 4, a region 5, a region 6, a region 7, a region 8 and a region 9; and
wherein the region 1 is selected from Chr3: 179450970-179451058 bp positive strand, the region 2 is selected from Chr3: 179451090-179451203 bp positive strand, the region 3 is selected from Chr3: 179451231-179451356 bp positive strand, the region 4 is selected from Chr3: 179451379-179451472 bp positive strand, the region 5 is selected from Chr3: 179451444-179451662 bp positive strand, the region 6 is selected from Chr3: 179451803-179451709 bp negative strand, the region 7 is selected from Chr3: 179451675-179451569 bp negative strand, the region 8 is selected from Chr3: 179451457-179451278 bp negative strand, the region 9 is selected from Chr3: 179451158-179451038 bp negative strand.

4. A detection reagent comprising a nucleic acid combination, wherein the nucleic acid combination is used for detecting methylation level of a target region in human GNB4 gene, and the target region comprises at least one CpG site and is selected from a full-length region or partial region of the following regions of GNB4 gene:
Chr3: 179450970-179451662 bp positive strand, and/or
Chr3: 179451803-179451038 bp negative strand.

5. A detection reagent for liver cancer diagnosis or auxiliary diagnosis, comprising a nucleic acid combination for detecting methylation level of a CpG island of human GNB4 gene, and the CpG island is selected from a full-length region or partial region of the following regions of GNB4 gene:
Chr3: 179450970-179451662 bp positive strand, and/or
Chr3: 179451803-179451038 bp negative strand.

6. The detection reagent according to claim 4 or 5, wherein the nucleic acid combination comprises primers capable of PCR-amplifying the target region and/or a probe capable of labeling the target region.

7. The detection reagent according to claim 4 or 5, wherein the target region according to claim 4, or the CpG island according to claim 5 is selected from a full-length region or partial region of at least one of the following regions of GNB4 gene: a region 1, a region 2, a region 3, a region 4, a region 5, a region 6, a region 7, a region 8 and a region 9; and
wherein the region 1 is selected from Chr3: 179450970-179451058 bp positive strand, the region 2 is selected from Chr3: 179451090-179451203 bp positive strand, the region 3 is selected from Chr3: 179451231-179451356 bp positive strand, the region 4 is selected from Chr3: 179451379-179451472 bp positive strand, the region 5 is selected from Chr3: 179451444-179451662 bp positive strand, the region 6 is selected from Chr3: 179451803-179451709 bp negative strand, the region 7 is selected from Chr3: 179451675-179451569 bp negative strand, the region 8 is selected from Chr3: 179451457-179451278 bp negative strand, and the region 9 is selected from Chr3: 179451158-179451038 bp negative strand.

8. The detection reagent according to claim 7, the nucleic acid combination is at least one selected from the group consisting of: a nucleic acid combination 1 for detecting the region 1, a nucleic acid combination 2 for detecting the region 2, a nucleic acid combination 3 for detecting the region 3, a nucleic acid combination 4 for detecting the region 4, a nucleic acid combination 5 for detecting the region 5, a nucleic acid combination 6 for detecting the region 6, a nucleic acid combination 7 for detecting the region 7, a nucleic acid combination 8 for detecting the region 8, and a nucleic acid combination 9 for detecting the region 9; and
the nucleic acid combination 1 has base sequences as set forth in SEQ ID NOs. 1 to 3, the nucleic acid combination 2 has base sequencesas set forth in SEQ ID NOs. 4 to 6, the nucleic acid combination 3 has base sequences as set forth in SEQ ID NOs. 7 to 9, the nucleic acid combination 4 has base sequences as set forth in SEQ ID NOs. 10 to 12, the nucleic acid combination 5 has base sequences as set forth in SEQ ID NOs. 13 to 15, the nucleic acid combination 6 has base sequences as set forth in SEQ ID NOs. 16 to 18, the nucleic acid combination 7 has base sequences as set forth in SEQ ID NOs. 19 to 21, the nucleic acid combination 8 has base sequences as set forth in SEQ ID NOs. 22 to 24, and the nucleic acid combination 9 has base sequences as set forth in SEQ ID NOs. 25 to 27.

9. The detection reagent according to claim 4 or 5, further comprising a reagent for detecting a methylation status of the CpG island of human GNB4 gene, the means for detecting the methylation status comprises at least one of: methylation-specific PCR, bisulfite sequencing, methylation-specific microarray, whole-genome methylation sequencing, pyrosequencing, methylation-specific high-performance liquid chromatography, digital PCR, methylations-specific high-resolution melting curve analysis, methylation-sensitive restriction enzyme digestion and flap endonuclease method.

10. A detection kit for liver cancer diagnosis or auxiliary diagnosis, including the detection reagent of any one of claims 4 to 9.

11. The detection kit for liver cancer diagnosis or auxiliary diagnosis according to claim 10, wherein a sample to be tested using the kit is a blood sample or tissue sample collected from an individual to be tested; and/or
the kit further comprises a buffer, dNTPs, a DNA polymerase and water

12. A method for detecting, diagnosing, or assisting in diagnosis of, liver cancer, including:
A) detecting, in a sample from an individual to be tested, a methylation level of a full-length region or partial region in the following target regions of GNB4 gene:
Chr3: 179450970-179451662 bp positive strand, and/or
Chr3: 179451803-179451038bp negative strand; and
B) analyzing a test result;
wherein, the target region comprises at least one CpG site.

13. The method according to claim 12, wherein the target region is selected from a full-length region or partial region of at least one of the following regions of GNB4 gene: a region 1, a region 2, a region 3, a region 4, a region 5, a region 6, a region 7, a region 8 and a region 9;
wherein the region 1 is selected from Chr3: 179450970-179451058 bp positive strand, the region 2 is selected from Chr3: 179451090-179451203 bp positive strand, the region 3 is selected from Chr3: 179451231-179451356 bp positive strand, the region 4 is selected from Chr3: 179451379-179451472 bp positive strand, the region 5 is selected from Chr3: 179451444-179451662 bp positive strand, the region 6 is selected from Chr3: 179451803-179451709 bp negative strand, the region 7 is selected from Chr3: 179451675-179451569 bp negative strand, the region 8 is selected from Chr3: 179451457-179451278 bp negative strand, and the region 9 is selected from Chr3: 179451158-179451038 bp negative strand.

14. The method according to claim 12, wherein for the detection in step A), a reagent specific for the methylation of the target region of human GNB4 gene is used;
preferably, the reagent comprises a nucleic acid combination for detecting the methylation of the target region in human GNB4 gene;
more preferably, the nucleic acid combination comprises primers capable of PCR-amplifying the target region and/or a probes capable of labelling the target region;
more preferably, the nucleic acid combination is at least one selected from the group consisting of: a nucleic acid combination 1 for detecting the region 1, a nucleic acid combination 2 for detecting the region 2, a nucleic acid combination 3 for detecting the region 3, a nucleic acid combination 4 for detecting the region 4, a nucleic acid combination 5 for detecting the region 5, a nucleic acid combination 6 for detecting the region 6, a nucleic acid combination 7 for detecting the region 7, a nucleic acid combination 8 for detecting the region 8, and a nucleic acid combination 9 for detecting the region 9; and the nucleic acid combination 1 has base sequences as set forth in SEQ ID NOs. 1 to 3, the nucleic acid combination 2 has base sequencesas set forth in SEQ ID NOs. 4 to 6, the nucleic acid combination 3 has base sequences as set forth in SEQ ID NOs. 7 to 9, the nucleic acid combination 4 has base sequences as set forth in SEQ ID NOs. 10 to 12, the nucleic acid combination 5 has base sequences as set forth in SEQ ID NOs. 13 to 15, the nucleic acid combination 6 has base sequences as set forth in SEQ ID NOs. 16 to 18, the nucleic acid combination 7 has base sequences as set forth in SEQ ID NOs. 19 to 21, the nucleic acid combination 8 has base sequences as set forth in SEQ ID NOs. 22 to 24, and the nucleic acid combination 9 has base sequences as set forth in SEQ ID NOs. 25 to 27.

15. Use of a liver cancer marker in preparing a liver cancer detection product, wherein the marker is a CpG site of GNB4 gene and a CpG site of Riplet gene.

16. The use according to claim 15, wherein the CpG site of GNB4 gene is derived from at least one of the following regions: a region 10 and a region 11, the region 10 is selected from Chr3:179450948-179451805 positive strand, and the region 11 is selected from Chr3:179451805-179450948 negative strand; and/or
the CpG site of Riplet gene is derived from at least one of the following regions: a region 12 and a region 13, the region 12 is selected from Chr17:30971029-30971588 positive strand, and the region 13 is selected from Chr17:30971588-30971029 negative strand.

17. A nucleic acid combination for liver cancer detection, comprising a nucleic acid combination 10 for detecting methylation of GNB4 gene and a nucleic acid combination 11 for detecting methylation of Riplet gene; and
the nucleic acid combination 10 comprises a pair of primers 1, the pair of primers 1 having base sequences that have at least 90% identity with base sequence as set forth in SEQ ID NOs. 25-26; and
the nucleic acid combination 11 comprises a pair of primers 2, the pair of primers 2 having base sequences that have at least 90% identity with base sequence as set forth in SEQ ID NOs. 42-43.

18. The nucleic acid combination for liver cancer detection according to claim 17, wherein the nucleic acid combination 10 further comprises a probe 1, the probe 1 having a base sequence that has at least 90% identity with a base sequence as set forth in SEQ ID NO. 27; and
the nucleic acid combination 11 further comprises a probe 2, the probe 2 having a base sequence that has at least 90% identity with a base sequence as set forth in SEQ ID NO. 44.

19. A kit comprising the nucleic acid combination for liver cancer detection of claim 17 or 18.

20. Use of the nucleic acid combination for liver cancer detection according to claim 17 or 18 or a kit according to claim 19 in detecting, diagnosing, or assisting in the diagnosis of, liver cancer.

21. A method for detecting, diagnosing, or assisting in diagnosis of, liver cancer, including:
a) detecting, in a sample from an individual to be tested, methylation of a CpG site of GNB4 gene and methylation of a CpG site of Riplet gene;
b) analyzing a test result.

22. The method according to claim 21, wherein the CpG site of GNB4 gene is derived from at least one of the following regions: a region 10 and a region 11, the region 10 is selected from Chr3: 179450948-179451805 positive strand, and the region 11 is selected from Chr3: 179451805-179450948 negative strand; and/or
the CpG site of Riplet gene is derived from at least one of the following regions: region 12 and region 13, the region 12 is selected from Chr17:30971029-30971588 positive strand, and the region 13 is selected from Chr17:30971588-30971029 negative strand.
